# EUROPEAN PATENT APPLICATION

(11) **EP 2 666 463 A1**
(43) Date of publication of application: **27.11.2013**
(21) Application number: 12168741.2
(22) Date of filing: 21.05.2012
(51) Int. Cl.: A61K 9/08, A61K 31/095, A61K 31/198, A61K 31/519, A61K 47/00

(54) **Stabilized liquid composition comprising pemetrexed**

(71) Applicant: Synthon BV, 6545 CM Nijmegen (NL)
(72) Inventor: The designation of the inventor has not yet been filed
(74) Representative: van der Sterren-Mol, Josephine

(57) **Abstract**

The present invention relates to a stabilized liquid pharmaceutical composition comprising pemetrexed or a pharmaceutically acceptable salt thereof, monothioglycerol, a] at least one non-ionogenic water soluble polymer and/or b] an edetate.

## Description

Pemetrexed is a common name for the compound N-[4-[2-(2-amino-4,7-dihydro-4-oxo-1H-pyrrolo [2,3-d]pyrimidin-5-yl)ethyl]benzoyl]-L-glutamic acid of formula (1)

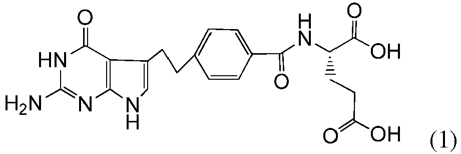

The compound has been first disclosed in US 5,344,932.

Pemetrexed is used in the treatment of malignant pleural mesothelioma and non-small cell lung cancer. The commercially-available product, sold, e.g., under brand name ALIMTA by Eli Lilly, comprises the disodium salt of pemetrexed as the active substance and is supplied in single-dose vials as a sterile lyophilized powder for intravenous administration.

While pemetrexed is administered to a patient in need thereof by intravenous infusion, it is not commercially available yet in the form of a ready to use solution. Such solution must presently be prepared immediately before use, by dissolving the content of the single-dose vial in water and diluting the obtained solution with an infusion solution. The relatively rapid formation of degradants is the main factor which has, so far, prevented aqueous pemetrexed formulations from being commercially available. According to WO 2012/015810, five major degradants of pemetrexed have been detected. Under acidic conditions, decarboxylation of glutamic acid is observed. Under alkaline conditions, degradation proceeds by side chain amide hydrolysis followed by deamination. In the presence of oxygen, two oxidative degradants result.

Attempts to prepare stable liquid compositions comprising pemetrexed have been made in the prior art. US Patent No. 6,686,365 (WO 01/56575) discloses ready to use (RTU) aqueous formulations of pemetrexed, which contain monothioglycerol, L-cysteine or thioglycolic acid as an antioxidant stabilizer. However, the later-published W02012/015810 provided evidence that the pemetrexed-containing formulations containing either monothioglycerol, L-cysteine or thioglycolic acid according to the US '365 patent, failed to demonstrate sufficient long term stability. For instance, an aqueous composition prepared according to Example 1 of US'365 comprising 40 mg/ml of pemetrexed disodium and 2.5 mg/ ml of monothioglycerol exhibited formation of 17.1 per cent total impurities when stored at 40°C for three months. Also, samples stored at 25°C showed precipitate at the end of six months of storage. Accordingly, said W02012/015810 proposed, as an improvement over US'365, a liquid composition comprising pemetrexed or a pharmaceutically acceptable salt thereof; an antioxidant such as lipoic acid, dihydrolipoic acid, methionine and mixtures thereof; a chelating agent such as lactobionic acid, tribasic sodium citrate, and mixtures thereof; and optionally up to about 75% propylene glycol. Such composition has been proven as being more stable than those of US'365.

There is, however, still a need to provide alternative ready-to-use liquid pharmaceutical formulations of pemetrexed that will have long term storage stability. It appeared from comparative experiments performed by the present inventors that monothioglycerol *per se* is the most effective antioxidant stabilizer among those disclosed in the prior art. However, based on teachings in the prior art, it is apparently not capable to assure long-term stability of an aqueous pemetrexed solution, particularly when stored at higher temperatures. Apparently, the nature of degradation pathways is quite complex and does not comprise only oxidation reactions. Thus, the presence of a co-stabilizer (or stability enhancer) would be advantageous. However, no suggestion for such co-stabilizer or stability enhancer of monothioglycerol was provided in any of the prior art documents.

Thus, it would be advantageous to provide a liquid pharmaceutical composition comprising pemetrexed and monothioglycerol, which would exhibit the desired stability during long term storage.

### SUMMARY OF THE INVENTION

The present invention relates to a stabilized liquid pharmaceutical composition comprising pemetrexed or a pharmaceutically acceptable salt thereof and monothioglycerol.

In one aspect, the present invention is drawn to an aqueous pharmaceutical composition comprising pemetrexed or a pharmaceutically acceptable salt thereof, monothioglycerol and a] at least one non-ionogenic water soluble polymer, advantageously a polyethylene glycol and/or a polyvinylpyrrolidone, and /or b] an edetate.

The concentration of the non-ionogenic polymer is advantageously in the range of 0.05 to 60% by weight.

The concentration of the edetate is advantageously in the range of 0.01 - 5 mg/ml, calculated as edetate disodium.

The pH of the composition is advantageously in the range of from about 8.0 to about10.0, preferably from about 8.6 to about 10.

In a particular aspect, the composition is free from propylene glycol or glycerol.

Further aspects of the present invention include the use of the above defined pemetrexed-comprising composition as a medicament, a vial comprising said composition, a kit or package form comprising said composition in a vial, and a process for preparing said composition.

One of the advantages of compositions of the invention is that they have sufficient long-term stability. The pemetrexed-comprising compositions of the invention advantageously exhibit less than 2 per cent of total impurities after at least about 3 months of storage at a temperature of 40°C, which indicates sufficient stability upon storage of about 18 months at 25°C.

The compositions of the invention are ready to use or can be further diluted with an infusion solution, such as e.g. a saline solution.

### DETAILED DESCRIPTION OF THE INVENTION

The present invention provides a stabilized aqueous pharmaceutical composition comprising pemetrexed or a pharmaceutically acceptable salt thereof and monothioglycerol; the composition is "stabilized" in comparison with the compositions comprising pemetrexed and monothioglycerol, which have been disclosed in the prior art. In particular, the compositions of the present invention, which are characterized by specific additives added to compositions comprising pemetrexed and monothioglycerol as stability enhancers, exhibit in stability studies at the same storage conditions generally less amount of impurities, particularly degradation products, than the same compositions without these additives.

In the most general aspect of the present invention, there are provided aqueous pharmaceutical compositions comprising pemetrexed or a pharmaceutically acceptable salt thereof and monothioglycerol, characterized in that the compositions further comprise, as stability enhancer(s), a] at least one non-ionogenic water soluble polymer, advantageously a polyethylene glycol and/or a polyvinylpyrolidone, and/or b] an edetate. Any of these both types of components may be used alone; however they may be also used together within the composition.

It should be understood that the non-ionogenic water soluble polymer is a pharmaceutically acceptable polymer, i.e. of proven and acceptable safety and quality.

The "polyethylene glycol" for use in accordance with the present invention is typically a liquid, low-molecular weight polyethylene glycol, e.g. a polyethylene glycol having a molecular weight of from 200 to 600.

The "polyvinylpyrrolidone", or "povidone" in short, for use in accordance with the present invention means a homopolymer of 1-vinyl-2-pyrrolidone of a polymerization degree resulting in a product, which is characterized by a viscosity in aqueous solution expressed as a K-value ranging from 10 to 120. See Kibbe, A.H. (Ed.), Handbook of Pharmaceutical Excipients, Third Edition, p. 433, for an explanation of the equation which is used for calculating the K-value.

Preferably, both polyethylene glycol and povidone are used of a quality prescribed for pharmaceutical use by acknowledged Pharmacopoeias, e.g. by US Pharmacopoeia (USP) or European Pharmacopoeia (Ph.Eur.). Advantageously, both polyethylene glycol and povidone are used as pyrogen-free products.

Other suitable non-ionogenic water soluble polymers may be a polypropylene glycol or a polyvinylalcohol.

The "edetate" for use in accordance with the present invention means edetic acid (ethylenediamino-tetraacetic acid) or a salt thereof. Typically, the disodium or tetrasodium salt of edetic acid (disodium or tetrasodium edetate) may be used as the starting material for making the compositions of the present invention. The actual form, in which the edetic acid is present in the invention composition, depends however on the pH of the solution and on the cation used both in the edetate and in the agent eventually used for adjusting the pH. Therefore, the general term "edetate" has been used and it includes any form of edetic acid actually present in the composition of the invention.

Pemetrexed or a pharmaceutically acceptable salt thereof is typically present in the composition of the present invention in concentrations of between 10 and 40 mg/ml, more preferably between 15 and 30 mg/ml, when calculated as anhydrous pemetrexed. In alternative aspects, the amount of pemetrexed may be outside these ranges but the amounts will be sufficient for single or multiple administrations of dosages generally regarded as effective amounts.

It is known in the art that the sodium (or disodium) salt of pemetrexed may exist, as a solid, in various hydrated forms. The pemetrexed free acid or other pemetrexed salts may also exhibit polymorphism. Any of such solid state forms are contemplated in the context of the present invention.

Monothioglycerol is typically present in compositions of the present invention in concentrations between 0.01 and 10 mg/ml, preferably between 1 and 10 mg/ml, more preferably between 1 and 5 mg/ml, particularly between 2 and 3 mg/ml.

The non-ionogenic water soluble polymer, if included in the compositions of the present invention, is typically present in concentrations between 0.05 and 60% by weight. The povidone may be preferably present in concentrations between 0.05 and 30% by weight; the polyethylene glycol may be preferably present in concentrations between 10 and 60% by weight.

The "edetate" as defined above, if included in the compositions of the present invention, is typically present in concentrations between 0.01 and 5 mg/ml, preferably between 0.05 and 2.5 mg/ml, more preferably between 0.1 and 1.5 mg/ml, when calculated as edetate disodium.

The liquid compositions of the present invention further include water as the solvent/ diluent. Water must be of pharmaceutically acceptable quality. Typically, water with the qualification "for injections", as defined in acknowledged Pharmacopoeias, is used.

For administration, water may comprise conventional tonicity agents assuring proper osmolality, for instance sodium chloride, mannitol etc, in suitable non-limiting concentrations, e.g. an aqueous saline solution.

Non-polymeric (monomeric) liquid co-solvents or co-diluents such as propylene glycol or glycerol are preferably not present in the compositions of the present invention.

In a particular embodiment of the present invention, the pH value of the stabilized liquid pharmaceutical composition of the present invention is from about 8.0 to about 10.0, preferably from about 8.6 to about 9.8, and more preferably from about 9.0 to about 9.6. Thus, to assure a proper pH value, a suitable pH adjusting agent may be present in the composition of the present invention. The suitable pH adjusting agent may be a pharmaceutically acceptable acid, base, salt or a combination thereof, e.g. an aqueous sodium hydroxide solution may be used to adjust the pH.

The compositions of the present invention may be prepared by a process comprising weighing the respective ingredients and dissolving them in water for injection, preferably under stirring. Advantageously, water is first deoxygenated by a suitable technique, e.g. by saturating it by an inert gas, by deaerating with ultrasound or in any other conventional way. The dissolution process is preferably conducted in an atmosphere of an inert gas such as nitrogen or argon. In the last step, the pH of the composition is adjusted to the desired value. The obtained solution is filtered and sterilized and filled into vials or similar container comprising the desired dose amount of pemetrexed per vial.

The suitability of compositions of the present invention has been studied and confirmed in stability studies. For purposes of the present invention, the impurities present in the pemetrexed-comprising formulations during stability studies were detected by high performance liquid chromatography (HPLC) equipped with a UV detector operating at a suitable wavelength (typically 227 nm). The amount of impurities was calculated on a normalized peak area response ("PAR") basis. As an acceptable limit demonstrating sufficient stability at the corresponding sampling point, the sum of peaks of all individual degradants in the invention compositions should not exceed 2% of the total PAR. The peak size of any individual degradant should not exceed 1% of the total PAR.

The results showing sufficient stability of the tested compositions for at least 3 months at 40°C are illustrated in Example 3 below.

A sufficient stability for 3 months at 40°C indicates the same sufficient stability for at least 18 months at 25°C.

Thus, the preferred compositions of the present invention are characterized in that they exhibit, after 3 months of storage in a closed container at 40°C, less than 2 per cent of total impurities. Alternatively, the preferred compositions of the present invention are characterized in that they exhibit, after 18 months of storage in a closed container at 25°C, less than 2 per cent of total impurities.

Accordingly, the temperatures at which the compositions of the present invention are kept for routine storage, within the period of the pharmaceutical shelf-life of the composition, are preferably room temperature or less (i.e., about 25°C or less). The compositions are preferably stored in tightly stoppered original containers and in the absence of light, typically closed dark glass vials. Under such conditions, the expected shelf-life of the compositions of the present invention is at least 18 months. While not required, it is contemplated that the shelf life can be further increased if the invention compositions are stored under refrigerated conditions, preferably at temperatures of less than about 10°C, more preferably between 2-8°C. It should be understood that the pemetrexed-comprising solutions remain in the temperature range described herein for substantially the entire period of storage before dilution and/or administration to the patient in need thereof.

Thus, in a specific aspect, the present invention includes a vial or similar container comprising a therapeutically effective dose amount of pemetrexed or a pharmaceutically acceptable salt thereof. The dose amount may comprise one or more doses of pemetrexed. Typically, vials may comprise pemetrexed disodium equivalent to 100 mg, 500 mg or 1000 mg of pemetrexed. Any suitable sterile vial or container fit for sterile storage of a pharmaceutical such as pemetrexed for extended periods of time may be used. Suitable containers can be glass vials, polypropylene or polyethylene vials or other special purpose containers.

A further aspect of the invention includes a kit or a package form for holding the pemetrexed-containing compositions described herein. The kit or package form contains at least one pharmaceutically acceptable vial or container containing one or more doses of pemetrexed-containing liquid formulations/compositions as well as other pharmaceutically necessary materials for storing and/or administering the drug, including instructions for storage and use, infusion bag or container with an infusion diluent, etc.

The compositions of the present invention and vials and kits comprising such compositions may be used as a medicament, particularly for treating a pemetrexed sensitive disease in mammals. Pemetrexed sensitive diseases include, but are not limited to, cancers, such as malignant pleural mesothelioma and non-small cell lung cancer. Thus, there are also provided methods of treating a pemetrexed sensitive disease in mammals. The use or methods include administering therapeutically effective amount of a pemetrexed-containing pharmaceutical composition as described hereinabove to a mammal in need thereof.

In a further aspect of the present invention, there is provided a process of stabilization, i.e. preventing formation of pemetrexed degradants, of liquid pemetrexed-containing pharmaceutical formulations during long term storage, particularly at room temperature. The process includes a step of dissolving the desired amount of pemetrexed or a pharmaceutically acceptable salt thereof in water further comprising monothioglycerol, at least one non-ionogenic water soluble polymer and/ or an edetate and adjusting pH of the solution to a value of from about 8.0 to about 10.0, preferably from 8.6 to about 9.8 and more preferably from about 9.0 to about 9.6.

The following examples serve to provide further appreciation of the claimed invention but are not meant in any way to restrict the scope of the present claims.

### EXAMPLES

### Example 1

Pemetrexed-containing compositions were prepared by dissolving, upon stirring, pemetrexed disodium salt (PXD) in a concentration of 20 mg/ml (calculated as pemetrexed) in water for injection under nitrogen atmosphere. Monothioglycerol (MTG) was added at a concentration of 2.4 mg/ml and disodium edetate (EDTA) and/or a polyvinylpyrrolidone (Kollidon 12) were added in amounts as indicated in Table 1 below. The pH was adjusted to 9.6 with 0.1N NaOH. The solution was filtered, filled into glass vials comprising 5 ml of the solution under nitrogen atmosphere, and vials were closed.

**Table 1: Compositions prepared according to Example 1**

| **PXD (mg/ml)** | **pH** | **MTG (mg/ml)** | **NaCl (mg/ml)** | **EDTA (mg/ml)** | **KOLLIDON 12 (mg/ml)** |
|---|---|---|---|---|---|
| 20 | 9.0 | 2.4 | - | 0.1 | - |
| 20 | 9.6 | 2.4 | - | 0.1 | - |
| 20 | 9.0 | 2.4 | - | 0.5 | - |
| 20 | 9.6 | 2.4 | - | 0.5 | - |
| 20 | 9.0 | 2.4 | - | 1.0 | - |
| 20 | 9.6 | 2.4 | - | 1.0 | - |
| 20 | 9.0 | 2.4 | 9 | 0.1 | - |
| 20 | 9.6 | 2.4 | 9 | 0.1 | - |
| 20 | 9.0 | 2.4 | 9 | 0.5 | - |
| 20 | 9.6 | 2.4 | 9 | 0.5 | - |
| 20 | 9.0 | 2.4 | 9 | - | 10 |
| 20 | 9.6 | 2.4 | 9 | - | 10 |

### Example 2

Pemetrexed-containing compositions were prepared by dissolving, upon stirring, pemetrexed disodium salt in a concentration of 25 mg/ml (calculated as pemetrexed) in a solvent system consisting of polyethylene glycol (PEG, MW 400) and water for injection under nitrogen atmosphere. Monothioglycerol was added at a concentration of 2.4 mg/ml and disodium edetate and/or polyvinylpyrrolidone was added in amounts as indicated in Table 2 below. The pH was adjusted to 9.6 with 0.1N NaOH. The solution was filtered, filled into glass vials comprising 4 ml of the solution under nitrogen atmosphere and the vials were closed.

**Table 2: Compositions prepared according to Example 2**

| **PXD (mg/ml)** | **pH** | **MTG (mg/ml)** | **NaCl (mg/ml)** | **PEG (% by weight)** | **KOLLIDON 12 (mg/ml)** |
|---|---|---|---|---|---|
| 25 | 9.6 | 2.4 | 9 | 40 | 10 |
| 25 | 9.6 | 2.4 | 9 | 40 | 1 |
| 25 | 9.6 | 2.4 | 9 | 40 | - |
| 25 | 9.6 | 2.4 | - | 40 | 1 |

### Example 3 - Stability study results

The compositions of the present invention were tested in a stability study at 40°C and at 25°C in closed glass vials. Impurities present in the pemetrexed-comprising formulations during stability studies were detected by high performance liquid chromatography (HPLC) equipped with a UV detector operating at 227 nm. The amount of impurities was calculated on a normalized peak area response ("PAR") basis.

As shown in W02012/015810, the comparative liquid composition comprising only 40 mg/ml pemetrexed and 2.5 mg/ ml of thioglycerol exhibited formation of 17.1 per cent total impurities after 3 months of storage at 40°C. To the contrary, the compositions in accordance with the invention show improved stability as can be seen in Tables 3-5 below, wherein PXD represents pemetrexed (charged as pemetrexed disodium), MTG is monothioglycerol, PEG is polyethylene glycol and EDTA is disodium edetate.

**Table 3: Stability results of compositions with an edetate**

| **PXD (mg/ml)** | **pH** | **MTG (mg/ml)** | **NaCl (mg/ml)** | **EDTA (mg/ml)** | **ΣIMP. START** | **Σ IMP 3 MONTHS 40°C** | **Σ IMP 6 MONTHS 40°C** | **Σ IMP 6 MONTHS 25°C** |
|---|---|---|---|---|---|---|---|---|
| 20 | 9.0 | 2.4 | - | 0.1 | 0.42 | 1.13 | 0.88 | 0.88 |
| 20 | 9.6 | 2.4 | - | 0.1 | 0.40 | 0.82 | 0.95 | 1.00 |
| 20 | 9.0 | 2.4 | - | 0.5 | 0.42 | 1.03 | 0.98 | 1.00 |
| 20 | 9.6 | 2.4 | - | 0.5 | 0.40 | 0.98 | 1.17 | 0.98 |
| 20 | 9.0 | 2.4 | - | 1.0 | 0.39 | 1.07 | 1.38 | 0.95 |
| 20 | 9.6 | 2.4 | - | 1.0 | 0.44 | 0.80 | 0.82 | 0.73 |
| 20 | 9.0 | 2.4 | 9 | 0.1 | 0.44 | 0.88 | 1.47 | 0.84 |
| 20 | 9.6 | 2.4 | 9 | 0.1 | 0.42 | 0.81 | 0.99 | 0.94 |
| 20 | 9.0 | 2.4 | 9 | 0.5 | 0.40 | 0.91 | 1.16 | 0.89 |
| 20 | 9.6 | 2.4 | 9 | 0.5 | 0.43 | 0.79 | 1.16 | 0.91 |
| 20 | 9.0 | 2.4 | 9 | 1.0 | 0.42 | 0.92 | 0.98 | 0.87 |
| 20 | 9.6 | 2.4 | 9 | 1.0 | 0.44 | 0.87 | 0.97 | 0.84 |

**Table 4: Stability results of compositions with a polyvinylpyrrolidone**

| **PXD (mg/ml)** | **pH** | **MTG (mg/ml)** | **KOLLIDON 12 (mg/ml)** | **Σ IMP. START** | **Σ IMP 3 MONTHS 40°C** | **Σ IMP 6 MONTHS 40°C** | **Σ IMP 6 MONTHS 25°C** |
|---|---|---|---|---|---|---|---|
| 20 | 9.0 | 2.4 | 10 | 0.53 | 1.01 | 1.11 | 0.85 |
| 20 | 9.6 | 2.4 | 10 | 0.48 | 0.82 | 0.79 | 0.79 |

**Table 5: Stability results of compositions with a polyethylene glycol, optionally in admixture with a polyvinylpyrrolidone**

| **PXD (mg/ml)** | **pH** | **MTG (mg/ml)** | **NaCl (mg/ml)** | **PEG (% by weight)** | **KOLLIDON 12 (mg/ml)** | **Σ IMP. START** | **Σ IMP 1 MONTH 40 °C** | **Σ IMP 3 MONTHS 40 °C** |
|---|---|---|---|---|---|---|---|---|
| 25 | 9.6 | 2.4 | 9 | 40 | 10 | 0.36 | 0.60 | 0.70 |
| 25 | 9.6 | 2.4 | 9 | 40 | 1 | 0.27 | 0.65 | 0.66 |
| 25 | 9.6 | 2.4 | 9 | 40 | - | 0.27 | 0.63 | 0.65 |
| 25 | 9.6 | 2.4 | - | 40 | 1 | 0.26 | 0.68 | 0.69 |

## Claims

1. An aqueous pharmaceutical composition comprising pemetrexed or a pharmaceutically acceptable salt thereof and monothioglycerol, **characterized in that** it further comprises a] at least one non-ionogenic water soluble polymer and /or b] an edetate.

2. The composition according to claim 1, wherein the non-ionogenic water soluble polymer is a polyethylene glycol, a polyvinylpyrrolidone, a polypropylene glycol or a polyvinylalcohol.

3. The composition according to claim 1 or 2, wherein the edetate is disodium edetate.

4. The composition according to any one of claims 1-3, wherein the concentration of the non-ionogenic water soluble polymer is in the range of 0.05 to 60% by weight.

5. The composition according to any one of claims 1-4, wherein the concentration of the edetate is in the range of 0.01 - 5 mg/ml, calculated as disodium edetate.

6. The composition according to any one of claims 1-5, wherein the pH is in a range of from about 8.0 to about10.0, preferably from about 8.6 to about 9.8, more preferably from about 9.0 to about 9.6.

7. A vial or similar container comprising the composition according to any one of claims 1-6 comprising a therapeutically effective dose amount of pemetrexed or a pharmaceutically acceptable salt thereof.

8. The vial according to claim 7, wherein the composition comprises a dose of pemetrexed disodium equivalent to 100 mg, 500 mg or 1000 mg of pemetrexed.

9. A kit or package form comprising a vial or similar container according to claim 7 or 8.

10. The composition according to any one of claims 1-6, a vial according to claim 7 or 8, or a kit or package form according to claim 9 for use as a medicament.

11. The composition, vial, or kit according to claim 10 for use in the treatment of malignant pleural mesothelioma and non-small cell lung cancer.

12. A process for preventing formation of pemetrexed degradants in a liquid pemetrexed-containing pharmaceutical formulation during long term storage, which includes a step of dissolving pemetrexed or a pharmaceutically acceptable salt thereof, monothioglycerol, at least one non-ionogenic water soluble polymer and/or an edetate in water and adjusting the pH of the solution to a value of from about 8.0 to about 10.0, preferably from 8.6 to about 9.8 and more preferably from about 9.0 to about 9.6.

13. A process for preparing the composition according to any one of claims 1-6, comprising dissolving pemetrexed or a pharmaceutically acceptable salt thereof, monothioglycerol, at least one non-ionogenic water soluble polymer and/or an edetate in water, and adjusting the pH of the solution to a value of from about 8.0 to about 10.0.
